# EUROPÄISCHE PATENTSCHRIFT

(11) **EP 1 309 815 B1**
(45) Veröffentlichungstag und Bekanntmachung des Hinweises auf die Patenterteilung: **26.10.2005**
(21) Anmeldenummer: 01947391.7
(22) Anmeldetag: 25.06.2001
(51) Int. Cl.: F16M 11/04, F16M 11/10

(54) **TRÄGER ZUM TRAGEN VON ZUMINDEST EINEM APPARAT**
SUPPORT FOR SUPPORTING AT LEAST ONE DEVICE
SUPPORT POUR AU MOINS UN APPAREIL

(30) Priorität: 17.08.2000 DE 10040343
(43) Veröffentlichungstag der Anmeldung: 14.05.2003
(73) Patentinhaber: Mavig GmbH, 81829 München (DE)
(72) Erfinder: KUHN, Peter, 81545 München (DE)
(74) Vertreter: Körfer, Thomas
(86) Internationale Anmeldenummer: PCT/EP2001/007161
(87) Internationale Veröffentlichungsnummer: WO 2002/014733

(56) Entgegenhaltungen:
- EP-A- 0 862 014
- CH-A- 653 464
- DE-C- 862 557
- US-A- 4 365 561
- US-A- 4 516 751

## Beschreibung

Die Erfindung betrifft einen Träger zum Tragen von zumindest einem Apparat, insbesondere zum Tragen von Bildschirmsicht- und anderen medizinischen Geräten.

Die bekannten Träger zum Tragen von Apparaten, insbesondere für den Einsatz in medizinischen Einrichtungen, dienen zum bodenfreien Befestigen eines Apparats mittels eines Federarms an der Decke eines Behandlungsraums oder dergleichen. Bekannt sind insbesondere Träger mit einem C-bogenförmigen Tragarm, die mittels einer Befestigungseinrichtung an dem Federarm befestigt sind. Der Aufhängepunkt, an dem der Tragarm mittels der Befestigungseinrichtung an dem Federarm befestigt ist, ist dabei durch die zu befestigenden Apparate vorgegeben, so daß bei der gewünschten horizontalen Ausrichtung des Tragarms der Schwerpunkt des belasteten Tragarms unter dem Aufhängepunkt liegt. Die besagten Apparate sind mittels eines an dem Tragarm befestigten Querträgers an dem Tragarm befestigt. Durch diese Befestigung ist die Neigung des Apparats relativ zu der Horizontalen bestimmt.

Der bekannte Träger hat mehrere Nachteile. Da die Neigung des Apparats fest vorgegeben ist, kann, was z.B. bei einem Bildschirm-Sichtgerät erforderlich ist, die Ausrichtung des Apparats nicht an den Blickwinkel eines Nutzers angepaßt werden. Außerdem ergibt sich bei Verwendung eines Apparats, der eine andere Schwerpunktverteilung und/oder ein anderes Gewicht als der Apparat aufweist, auf den der Träger abgestimmt ist, das Problem, daß der Träger in eine Stellung mit einer gegenüber der gewünschten Neigung geänderten Neigung geschwenkt wird, so daß die Neigung des befestigten Apparats ungünstig ist. Beispielsweise ergibt sich dann bei einem Bildschirm-Sichtgerät für alle bzw. für zumindest die meisten Nutzer ein ungünstiger Betrachtungswinkel.

Ein weiterer Nachteil, der sich insbesondere ergibt, wenn mehrere Apparate von dem Träger getragen werden, ist, daß die Stellung des Apparats bezüglich seiner vertikalen Achse relativ zu dem Träger fest vorgegeben ist. Insbesondere bei mehreren Apparaten ist es wünschenswert, daß diese zueinander gedreht sind, um den Zugang zu den Apparaten, z.B. zum Betätigen eines Schalters, zu erleichtern und eine vorteilhafte Blickrichtung auf jeden der Apparate, z.B. zum Ablesen von Informationen, zu optimieren.

Ein derartiger Träger ist beispielsweise aus der EP 0 862 014 A bekannt. Die Druckschrift offenbart eine Vorrichtung zur vorzugsweise wandgebundenen Unterstützung eines Fernsehers oder dergleichen. Die Vorrichtung umfaßt ein Gelenk, welches die Ausrichtung des Fernsehers in vertikaler und horizontaler Richtung erlaubt. Ein Stift definiert eine vertikale Achse, um welche Stützglieder relativ zueinander drehbar sind. Eine horizontale Achse ist zwischen Vorsprüngen vorgesehen. Die Position wird durch eine Schraube eingestellt, die durch ein Daumenrad betätigbar ist.

Der Erfindung liegt daher die Aufgabe zugrunde, einen Träger zu schaffen, bei dem die Orientierung eines von dem Träger getragenen Apparats bezüglich des Trägers und insbesondere die Neigung des Apparats einstellbar ist.

Die Aufgabe wird durch die Merkmale des Anspruchs 1 gelöst. Vorteilhafte Weiterbildungen der in Anspruch 1 angegebenen Erfindung sind durch die in den Unteransprüchen aufgeführten Maßnahmen möglich.

Der erfindungsgemäße Träger hat den Vorteil, daß die Neigung des von dem Träger getragenen Apparats einstellbar ist. Dadurch kann z.B. ausgehend von einer horizontalen Orientierung des Apparats eine Drehung aus der Horizontalen zum Neigen des Apparates erfolgen. Dadurch kann die Ausrichtung des Apparats an einen Nutzer angepaßt werden, um z.B. einen vorteilhaften Zugriff oder eine Anpassung an die Blickrichtung des Nutzers zu ermöglichen.

In vorteilhafter Weise ist die Trageplatte in einem ersten Lager der Tragevorrichtung drehbar gelagert, wobei das erste Lager die Drehachse definiert, um die die Trageplatte zum Neigen gedreht wird. Dabei ist es außerdem vorteilhaft, daß die Trageplatte ferner in einem zweiten Lager der Tragevorrichtung gelagert ist, wobei das zweite Lager mittels des Stellglieds zum Neigen der Trageplatte räumlich verstellbar ist. Durch das erste Lager wird eine relativ zu der Tragevorrichtung ortsfest angeordnete Drehachse definiert, um die die Trageplatte zum Neigen drehbar ist. Die Drehachse kann dabei auch außerhalb der Trageplatte liegen. Bei festgelegter Drehachse bleibt für die Trageplatte ein weiterer Freiheitsgrad, der in der Drehung um diese Achse besteht. Durch das zweite Lager wird auch dieser verbleibende Freiheitsgrad festgelegt, wobei durch Verstellen des Stellglieds zum Neigen der Trageplatte eine Drehung der Trageplatte um die Drehachse erfolgt. Dadurch ist eine zumindest im wesentlichen spielfreie Lagerung der Trageplatte durch die Tragevorrichtung gegeben, wobei die Drehstellung der Trageplatte in beiden Drehrichtungen bezüglich der Drehachse durch die Lagerung festgelegt ist.

In vorteilhafter Weise weist das zweite Lager zumindest ein Pleuel auf, das einerseits mit einem Lagerelement der Trageplatte und andererseits mit einem Lagerelement der Tragevorrichtung verbunden ist. Durch das Pleuel ist eine einfache Übertragung der von dem Stellglied vermittelten Verstellung auf die Trageplatte gegeben, wobei bei der Verstellung eine Verschiebung des Lagerelements der Trageplatte relativ zu dem Lagerelement der Tragevorrichtung, insbesondere in einer horizontalen Richtung, ausgeglichen wird.

Vorteilhaft ist es, daß das Stellglied das zweite Lager in einer Richtung verstellt, die zumindest im wesentlichen senkrecht zu der Tragefläche der Trageplatte ist. Dadurch können die erzeugten und auf die Bauteile des Trägers, insbesondere auf das zweite Lager, einwirkenden Kräfte auf das erforderliche Maß reduziert werden. Außerdem erfolgt eine vorteilhafte Hubübertragung der Verstellung des Stellglieds in die Neigung der Trageplatte.

Vorteilhaft ist es, daß das Stellglied eine Handkurbel zum Betätigen des Stellglieds umfaßt. Dadurch läßt sich das Stellglied schnell, einfach und präzise verstellen, um die gewünschte Neigung des Apparats, der auf der Trageplatte angeordnet ist, zu erreichen.

In vorteilhafter Weise umfaßt das Stellglied eine mittels der Handkurbel betätigbare Spindel, die eine Rotationsbewegung der Handkurbel in einen Hub zum Neigen der Trageplatte umsetzt. Dabei läßt sich über die Ganghöhe der Spindel das Übersetzungsverhältnis, d.h. der Hub zum Neigen der Trageplatte für eine von der Handkurbel erwirkbare Umdrehung der Spindel, vorgeben, so daß bei einer geringen Ganghöhe des Gewindes der Spindel eine besonders genaue und bei einer großen Ganghöhe der Spindel eine besonders schnelle Veränderung der Neigung der Trageplatte erreichbar ist. Die Genauigkeit der Einstellung wird dabei außerdem von dem Durchmesser der Kreislinie beeinflußt, auf der die Kurbel der Handkurbel beim Betätigen der Spindel bewegbar ist.

In vorteilhafter Weise weist die Tragevorrichtung ein drittes Lager zum Schwenken der Trageplatte um eine Achse auf, die zumindest näherungsweise senkrecht zu der Tragefläche der Trageplatte ist. Dadurch kann zusätzlich zu der Neigung der Trageplatte, die durch das erste und zweite Lager gegeben sein kann, eine Drehung der Trageplatte durch das dritte Lager ermöglicht werden, so daß eine Drehung des Apparats um zwei zueinander senkrechte Drehachsen ermöglicht wird. Dabei ist es besonders vorteilhaft, daß die durch das dritte Lager gegebene Drehachse zumindest näherungsweise senkrecht zu der Tragefläche der Trageplatte bzw. näherungsweise horizontal ist. Dadurch kann eine ergonomische Anpassung der Ausrichtung des Apparats, insbesondere eine Anpassung der eingestellten Blickrichtung, an einen Benutzer erfolgen.

Dabei ist es ferner vorteilhaft, daß eine Bremsvorrichtung zum Dämpfen der durch das dritte Lager gegebenen Lagerung der Trageplatte vorgesehen ist. Dadurch wird verhindert, daß versehentlich, z.B. bei einer Bewegung des Trägers oder bei einer Betätigung des Apparats, eine Verschwenkung erfolgt. Ferner kann durch Feststellen der Bremsvorrichtung eine Arretierung der Verschwenkung der Trageplatte bzw. des auf der Trageplatte angeordneten Apparats bezüglich der Achse des dritten Lagers erfolgen.

Die Erfindung wird nachfolgend anhand eines bevorzugten Ausführungsbeispiels unter Bezugnahme auf die Zeichnung näher beschrieben. In der Zeichnung zeigen:
- Fig. 1: eine Seitenansicht eines Trägers;
- Fig. 2: eine Vorderansicht des in Fig. 1 dargestellten Trägers;
- Fig. 3: den in Fig. 1 mit III bezeichneten Ausschnitt gemäß dem bevorzugten Ausführungsbeispiel der Erfindung in einer teilweisen Schnittdarstellung; und
- Fig. 4: den in Fig. 3 dargestellten Ausschnitt des bevor- zugten Ausführungsbeispiels in einer teilweisen Schnittdarstellung in einer Ansicht, die durch die in Fig. 3 mit IV bezeichnete Richtung gegeben ist.

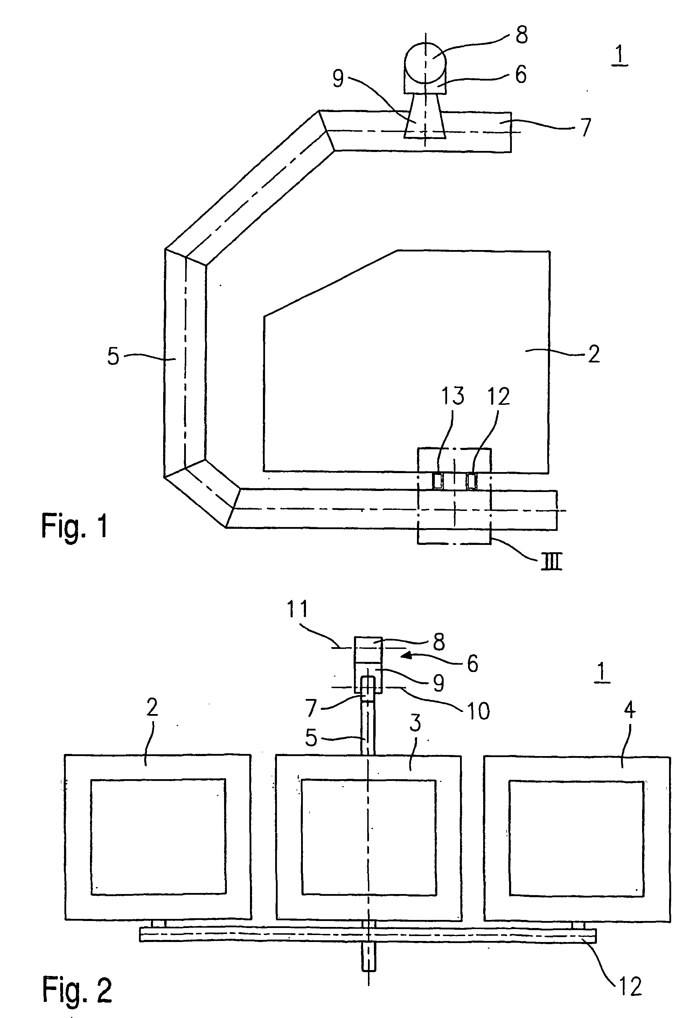

Fig. 1 zeigt den grundsätzlichen Aufbau eines Trägers 1 zum Tragen von Apparaten 2, 3, 4, insbesondere zum Tragen von Bildschirm-Sichtgeräten und anderen medizinischen Geräten. Der Träger 1 dient insbesondere zur Verwendung in ärztlichen Einrichtungen, wie Arztpraxen, Klinikräumen und Operationssälen, um eine bodenfreie Trage- bzw. Befestigungseinrichtung für die medizinischen Geräte und dergleichen bereitzustellen. Der erfindungsgemäße Träger 1 eignet sich jedoch auch für andere Anwendungsfälle.

Der Träger 1 weist einen C-bogenförmigen Tragarm 5 und eine mit diesem verbundene Befestigungseinrichtung 6 auf, wobei die Befestigungseinrichtung 6 am oberen Teil 7 des Tragarms 5 angeordnet ist. Die Befestigungseinrichtung 6 umfaßt ein Gelenkstück 8, an dem der Träger 1 mit einer geeigneten Haltevorrichtung verbindbar ist. Die Haltevorrichtung kann z.B. ein an der Decke des Raumes, in dem der Träger zum Einsatz kommt, befestigter Federarm sein. Die Befestigungseinrichtung 6 umfaßt außerdem ein Verbindungsstück 9, an dem die Befestigungseinrichtung 6 mit dem oberen Teil 7 des Tragarms 5 verbunden ist. Die Verbindung kann dabei zumindest in gewissen Grenzen eine Drehung des Tragarms 5 um die in Fig. 2 dargestellte Achse 10 relativ zu dem Verbindungsstück 9 der Befestigungseinrichtung 6 erlauben, oder auch starr ausgebildet sein, so daß die Drehbarkeit bezüglich der Achse 10 verhindert ist. Jedenfalls ist der Tragarm 5 an dem Gelenkstück 8 bezüglich der Haltevorrichtung um die Achse 11, die die Drehachse des Gelenkstücks 8 darstellt, drehbar gelagert, so daß der Schwerpunkt des mit den Apparaten 2, 3, 4 belasteten Trägers 1 bezüglich seiner vertikalen Richtung unterhalb der Achse 11 zum Liegen kommt.

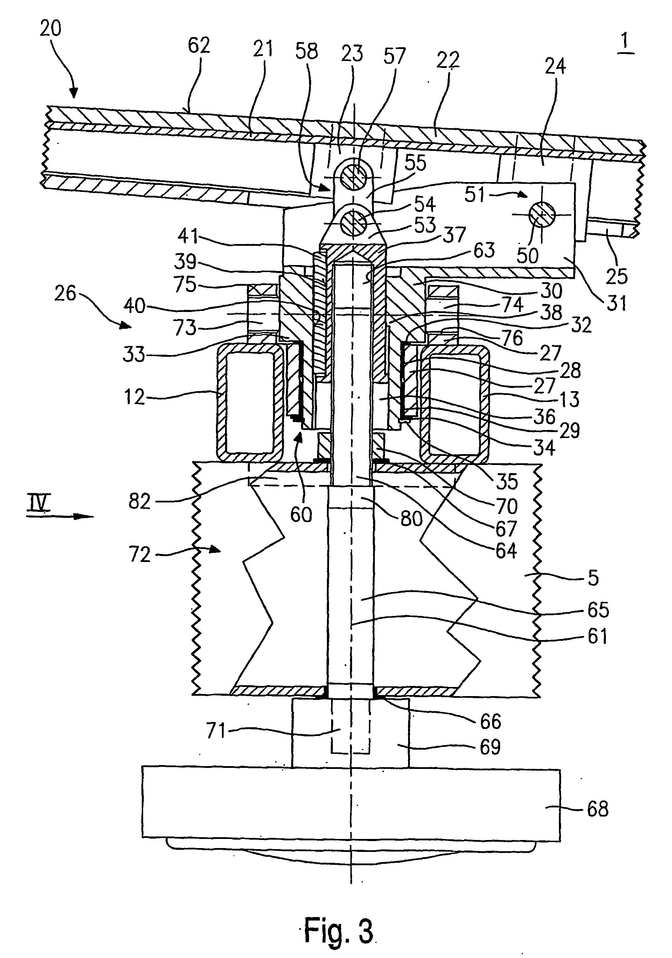

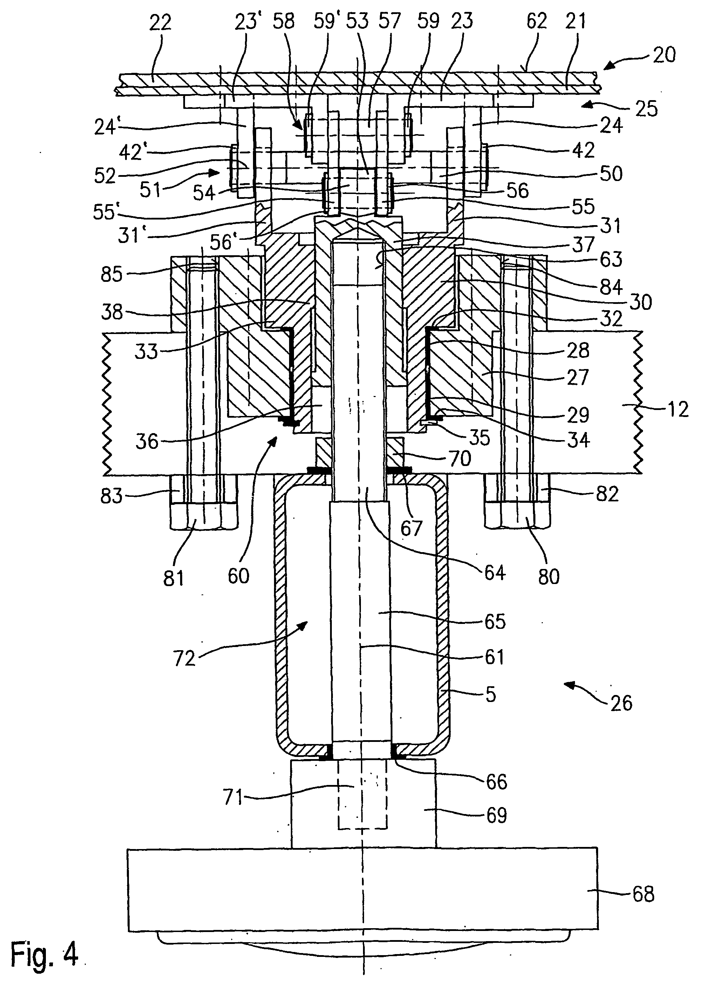

Die Apparate 2, 3, 4 sind an aus Hohlprofilkörpern gebildeten Querträgern 12, 13 mit rechteckigem Querschnitt befestigt. Die Befestigung wird nachfolgend anhand der Fig. 3 und 4 näher beschrieben. Die Querträger 12, 13 sind mit dem Tragarm 5 verbunden, wobei die Verbindung eine Schweißverbindung, Schraubverbindung oder dergleichen sein kann.

Fig. 2 zeigt den in Fig. 1 dargestellten Träger 1 in einer Vorderansicht. Bereits beschriebene Elemente sind in der Fig. 2 und in allen anderen Figuren mit übereinstimmenden Bezugszeichen versehen, wodurch sich eine wiederholende Beschreibung erübrigt.

Fig. 3 zeigt den in Fig. 1 mit III bezeichneten Ausschnitt gemäß einem bevorzugten Ausführungsbeispiel der Erfindung.

Der Träger 1 weist eine Trageplatte 20 auf, die einen vorzugsweise aus Metall ausgebildeten Grundkörper 21 und einen vorzugsweise aus Kunststoff ausgebildeten den Grundkörper 21 umhüllenden Hüllkörper 22 umfaßt. Die Trageplatte 20 weist außerdem Lagerelemente 23, 24 auf, die mit dem Grundkörper 21, z.B. durch eine Schweißverbindung, verbunden sind. Der Hüllkörper 22 der Trageplatte 20 weist eine Aussparung 25 auf, durch die die Lagerelemente 23, 24 zum Verbinden mit einer Tragevorrichtung 26 zugänglich sind.

Die Tragevorrichtung 26 weist einen Grundkörper 27 auf, der mit den Querträgern 12, 13 verbunden ist. In dem Grundkörper 27 der Tragevorrichtung 26 ist mittels der Lagerhülsen 28, 29 ein Lagerkörper 30 gelagert, der ein Lagerelement 31 umfaßt. Die Lagerhülse 28 weist einen Bund 32 auf, der zwischen dem Grundkörper 27 und einem Vorsprung 33 des Lagerkörpers 30 angeordnet ist, wodurch neben der radialen Lagerung durch die Lagerhülsen 28, 29 eine axiale Lagerung gegeben ist. Dabei ist auch an der Lagerhülse 29 ein Bund 34 ausgebildet, der zumindest teilweise zwischen einem Sicherungsring 35 und dem Grundkörper 27 vorgesehen ist, so daß in Zusammenwirkung mit der Lagerhülse 28 eine zweiseitige axiale Lagerung gegeben ist. Dabei wird durch den Sicherungsring 35 der Lagerkörper 30 gesichert, wodurch eine Befestigung des Lagerkörpers 30 mittels des Grundkörpers 27 an die Querträger 12, 13 gegeben ist.

Der Lagerkörper 30 weist eine Stufenbohrung 36 auf, in der eine Spindelbuchse 37 geführt ist, wobei die Bewegbarkeit der Spindelbuchse 37 in der Führung durch einen an dem Lagerkörper 30 ausgebildeten Anschlag 38 begrenzt ist. Die Stufenbohrung 36 weist eine schlitzartige Aussparung 39 auf, der teilweise eine ebenfalls schlitzartige Aussparung 40, die in dem Lagerkörper 30 ausgebildet ist, gegenüberliegt. Zwischen dem Lagerkörper 30 und der Spindelbuchse 37 ist ein Zwischenstück 41 vorgesehen, das teilweise in die schlitzartige Aussparung 39 der Spindelbuchse 37 und teilweise in die schlitzartige Aussparung 40 des Vorsprungs 33 eingreift, so daß eine Drehbewegung der Spindelbuchse 37 relativ zu dem Lagerkörper 30 verhindert ist. Das Zwischenstück 41 kann aus einem Federblech oder einem Metallblättchen ausgebildet sein. Da die Aussparung 40 in dem Lagerkörper 30 längs der gesamten Stufenbohrung 36 ausgebildet und insbesondere an ihren Enden offen ist, wird die axiale Verschiebbarkeit der Spindelbuchse 37 durch den beschriebenen Mechanismus, der ein Verdrehen der Spindelbuchse 37 bezüglich des Lagerkörpers 30 verhindert, nicht beeinflußt.

Der erfindungsgemäße Träger gemäß dem bevorzugten Ausführungsbeispiel wird im folgenden unter zusätzlicher Bezugnahme auf Fig. 4 näher beschrieben. Dabei zeigt Fig. 4 den in Fig. 3 dargestellten und in Fig. 1 mit III bezeichneten Ausschnitt aus der in Fig. 3 mit IV bezeichneten Richtung.

Dem Lagerelement 31 des Lagerkörpers 30 liegt ein Lagerelement 31' des Lagerkörpers 30 gegenüber. Entsprechend liegt dem Lagerelement 24 der Trageplatte 20, das mit dem Grundkörper 21 der Trageplatte 20 verbunden ist, das ebenfalls mit dem Grundkörper 21 der Trageplatte 20 verbundene Lagerelement 24' gegenüber. Der Lagerkörper 30 ist an den Lagerelementen 31, 31' mittels eines Lagerbolzens 50 mit den Lagerelementen 24, 24' der Trageplatte 20 verbunden, wobei der Lagerbolzen 50 bezüglich der Lagerelemente 24, 24' der Trageplatte 20 und/oder bezüglich der Lagerelemente 31, 31' des Lagerkörpers 30 drehbar ist. Dabei ist der Lagerbolzen 50 mittels der Sicherungsringe 42, 42' gegen eine axiale Verschiebung gesichert.

Durch die Lagerelemente 31, 31' des Lagerkörpers 30, die Lagerelemente 24, 24' der Trageplatte 20 und den Lagerbolzen 50 ist ein erstes Lager 51 gebildet, das eine mit der Achse des Lagerbolzens 50 übereinstimmende Drehachse 52 definiert, um die die Trageplatte 20 drehbar ist. Somit ist die Trageplatte 20 in dem ersten Lager 51 der Tragevorrichtung 26 drehbar gelagert, wobei die Drehachse durch die Achse des Lagerbolzens 50 gegeben ist.

Die Spindelbuchse 37 weist das Lagerelement 53 auf, das mittels des Lagerbolzens 54 mit dem Pleuel 55 und dem Pleuel 55' verbunden ist, wobei der Lagerbolzen 54 gegenüber dem Lagerelement 53 und/oder den Pleuel 55, 55' drehbar ist, und der Lagerbolzen 54 mit den Sicherungsringen 56, 56' gegen ein axiales Verschieben gesichert ist. Daher sind die Pleuel 55, 55' einerseits mittels des Lagerbolzens 54 mit dem Lagerelement 53 der Spindelbuchse 37 der Tragevorrichtung 26 verbunden. Andererseits sind die Pleuel 55, 55' mittels eines drehbaren Lagerbolzens 57 mit den Lagerelementen 23, 23', die mit dem Grundkörper 21 der Trageplatte 20 verbunden sind, verbunden. Durch das Lagerelement 53 der Tragevorrichtung 26, die Lagerelemente 23, 23' der Trageplatte 20 und die Pleuel 55, 55', die einerseits mittels des Lagerbolzens 54 mit dem Lagerelement 53 der Tragevorrichtung 26 und andererseits mittels des Lagerbolzens 57 mit den Lagerelementen 23, 23' der Trageplatte 20 verbunden sind, ist ein zweites Lager 58 der Tragevorrichtung 26 gebildet, in dem die Trageplatte 20 zusätzlich zu der Lagerung in dem ersten Lager 51 gelagert ist. Dabei ist der Lagerbolzen 57 mit den Sicherungsringen 59, 59' in axialer Richtung gesichert.

Außerdem bildet der Lagerkörper 30 mit dem Grundkörper 27, den Lagerhülsen 28, 29 und dem Sicherungsring 35 ein drittes Lager 60, das Bestandteil der Tragevorrichtung 26 ist. Durch das dritte Lager 60 wird eine Drehbarkeit des Lagerkörpers 30 um eine Achse 61 ermöglicht, so daß die Trageplatte 20 um die Achse 61 drehbar ist, um die Trageplatte 20 zu schwenken. Dabei ist die Achse 61 zumindest näherungsweise senkrecht zu der Tragefläche 62 der Trageplatte 20 orientiert.

Die Spindelbuchse 37 weist eine Gewindebohrung 63 auf, in die ein Gewindeabschnitt 64 einer Spindel 65 eingreift. Die Spindel 65 durchdringt den Tragarm 5, wobei sie an dem Tragarm 5 mittels einer Lagerhülse 66 und mittels eines Lagerrings 67 gelagert ist. Dabei ist die Spindel 65 mit einer Handkurbel 68 verbunden, die ein Distanzstück 69 umfaßt, an dem sich die Lagerhülse 66 abstützt. Ein auf den Gewindeabschnitt 64 der Spindel 65 geschraubtes Einstellelement 70 dient zum Einstellen des Lagerspiels des durch die Lagerhülse 66, den Lagerring 67, den Tragarm 5 und das Distanzstück 69 gegebenen Lagers.

Die Handkurbel 68 ist an dem Distanzstück 69 mit einem Verbindungsstück 71 der Spindel 65 verbunden. Anstelle der Handkurbel 68 kann auch eine andere Verstellvorrichtung an dem Verbindungsstück 71 der Spindel 65 vorgesehen werden.

Die Spindel 65 bildet mit der Spindelbuchse 37 und der Handkurbel 68 ein Stellglied 72, das einen Hub zum Verstellen der Neigung der Trageplatte 20, insbesondere der Tragefläche 62 der Trageplatte 20, erzeugt. Der Hub des Stellglieds 72 wird dabei über das erste Lager 51 und das zweite Lager 58 in eine Drehbewegung bzw. Neigung der Trageplatte 20 umgesetzt. Durch Betätigen der Handkurbel 68 wird die Spindel 65 (zusammen mit dem Einstellelement 70) um die Achse 61 gedreht, so daß sich insbesondere der Gewindeabschnitt 64 der Spindel 65 relativ zu der mittels des Zwischenstücks 41 festgehaltenen Spindelbuchse 37 dreht, wodurch eine Verstellung bzw. ein Hub der Spindelbuchse 37 in einer axialen Richtung, die parallel zu der Achse 61 ist, erzeugt wird. Dadurch wird das zweite Lager 58 relativ zu dem ersten Lager 51 verstellt, wobei das erste Lager 51 die Drehachse 52 für eine Drehung bzw. Neigung der Trageplatte 20 vorgibt. Somit wird der von dem Stellglied 72 erzeugte Hub in eine Drehung der Trageplatte 20 um die Drehachse 52 umgesetzt.

Der Lagerkörper 30 der Tragevorrichtung 26 ist mittels des dritten Lagers 60 um die Achse 61 der Spindel 65 drehbar, wobei eine die Bremsschrauben 73, 74 umfassende Bremsvorrichtung zum Dämpfen der durch das dritte Lager 60 gegebenen Lagerung vorgesehen ist. Die Bremsschrauben 73, 74 sind in Gewindebohrungen 75, 76 des Grundkörpers 27 eingeschraubt, wobei durch die Einschraubtiefe die Reibungskraft, die die Stärke der Dämpfung vorgibt, einstellbar ist. Hierfür ist in vorteilhafter Weise an den dem Lagerkörper 30 zugewandten Enden der Bremsschrauben 73, 74 eine Reibungseinlage, die z.B. aus einer Gummimischung besteht, vorgesehen.

Die Tragefläche 62 dient zum Tragen des Apparats 3. Dabei kann der Apparat 3, z.B. durch Schraub- oder Klemmverbindungen, mit der Trageplatte 20 verbunden werden. Der Apparat 3 kann allerdings auch ohne eine weitere Verbindung auf die Tragefläche 62 der Trageplatte 20 gestellt werden. Durch das Stellglied 72 der Tragevorrichtung 26 kann die Neigung des Apparats 3 bezüglich der Horizontalen verstellt werden. Außerdem kann der Apparat 3 mittels der Tragevorrichtung 26 um die Achse 61, die näherungsweise vertikal orientiert ist, geschwenkt werden. Dadurch kann der Apparat 3 um zwei zumindest näherungsweise zueinander senkrechte Achsen, nämlich die Achse 61 und die Drehachse 52, gedreht werden.

Der Grundkörper 27 wird zum Befestigen an dem Tragarm 5 mittels der Befestigungsschrauben 80, 81 festgeschraubt, wobei sich die Befestigungsschrauben 80, 81 an Befestigungsplatten 82, 83 abstützen. Die Befestigungsplatten 82, 83 liegen dabei jeweils an der Unterseite des Querträgers 12 und an der Unterseite des Querträgers 13 an. Die Befestigungsschrauben 80, 81 sind dabei durch Aussparungen in den Befestigungsplatten 82, 83 geführt und zwischen den Querträgern 12, 13 angeordnet, wobei sie an ihren Enden in an dem Grundkörper 27 ausgebildete Gewindebohrungen 84, 85 eingreifen.

Die anhand der Fig. 3 und 4 beschriebene Befestigung der Tragevorrichtung 26 an den Querträgern 12, 13 ist vorzugsweise für die mittlere Position an den Querträgern 12, 13, d.h. für den Apparat 3, vorgesehen, da dort der Tragarm 5 unterhalb der Querträger 12, 13 angeordnet ist. Für die anderen Positionen, d.h. für die Apparate 2, 4 ist vorzugsweise eine etwas abweichende Befestigung vorgesehen. Da in diesem Fall der Tragarm 5 entfällt, wird anstelle der Befestigungsplatten 82, 83 eine einzelne große Befestigungsplatte verwendet, in die die Befestigungsschrauben 80, 81 an gleicher Stelle wie in Fig. 4 eingeschraubt werden, wobei die Befestigungsplatte zugleich eine Aussparung aufweist, durch die die Spindel 65 geführt ist und in der die Lagerhülse 66 angeordnet ist. Dadurch kann außerdem der Abstand zwischen dem Distanzstück 69 und dem Gewindeabschnitt 64 der Spindel 65 erheblich reduziert werden.

## Patentansprüche

1. Träger (1) zum Tragen von zumindest einem Apparat (2, 3, 4), insbesondere zum Tragen von Bildschirmsicht- und anderen medizinischen Geräten, mit einem Tragarm (5) und zumindest einer Tragevorrichtung (26), die zumindest mittelbar mit dem Tragarm (5) verbunden ist, wobei die Tragevorrichtung (26) zum Aufnehmen des Apparates (2, 3, 4) mit einer Trageplatte (20) verbunden ist, die mittels eines Stellglieds (72) zum Neigen des Apparats (2, 3, 4) neigbar ist,
wobei die Trageplatte (20) in einem ersten Lager (51) der Tragevorrichtung (26) drehbar gelagert ist, wobei das erste Lager (51) die Drehachse (52) definiert, um die die Trageplatte (20) zum Neigen drehbar ist,
und wobei die Trageplatte (20) ferner in einem zweiten Lager (58) der Tragevorrichtung (26) gelagert ist, wobei das zweite Lager (58) mittels des Stellglieds (72) zum Neigen der Trageplatte (20) räumlich verstellbar ist,
**dadurch gekennzeichnet,**
**daß** das zweite Lager (58) zumindest ein Pleuel (55) aufweist, das einerseits mit einem Lagerelement (23, 23') der Trageplatte (20) und andererseits mit einem Lagerelement (53, 53') der Tragevorrichtung (26) verbunden ist.

2. Träger nach Anspruch 1,
**dadurch gekennzeichnet,**
**daß** das Stellglied (72) das zweite Lager (58) in einer Richtung verstellt, die zumindest im wesentlichen senkrecht zu der Tragefläche (62) der Trageplatte (20) ist.

3. Träger nach Anspruch 1 oder 2,
**dadurch gekennzeichnet,**
**daß** das Stellglied (72) eine Handkurbel (68) zum Betätigen des Stellglieds (72) umfaßt.

4. Träger nach Anspruch 3,
**dadurch gekennzeichnet,**
**daß** das Stellglied (72) eine mittels der Handkurbel (68) betätigbare Spindel (65) umfaßt, die eine Rotationsbewegung der Handkurbel (68) in einen Hub zum Neigen der Trageplatte (20) umsetzt.

5. Träger nach einem der Ansprüche 1 bis 4,
**dadurch gekennzeichnet,**
**daß** die Tragevorrichtung (26) ein drittes Lager (60) zum Schwenken der Trageplatte (20) um eine Achse (61), die zumindest näherungsweise senkrecht zu der Tragefläche (62) der Trageplatte (20) ist, aufweist.

6. Träger nach Anspruch 5,
**dadurch gekennzeichnet,**
**daß** eine Bremsvorrichtung (73, .74) zum Dämpfen der durch das dritte Lager (60) gegebenen Lagerung der Trageplatte (20) vorgesehen ist.

## Claims

1. Support (1) for supporting at least one apparatus (2, 3, 4), in particular for supporting visual display units and other medical devices, comprising a support arm (5) and at least one support device (26), which is connected at least indirectly to the support arm (5), wherein the support device (26) for receiving the apparatus (2, 3, 4) is connected to a support plate (20), which is inclinable by means of a control element (72) for inclining the apparatus (2, 3, 4),
wherein the support plate (20) is mounted rotatably in a first bearing (51) of the support device (26),
wherein the first bearing (51) defines the axis of rotation (52), about which the support plate (20) is rotatable for the purpose of inclination,
and wherein the support plate (20) is further mounted in a second bearing (58) of the support device (26),
wherein the second bearing (58) is three-dimensionally adjustable by means of the control element (72) for inclining the support plate (20),
**characterized in**
**that** the second bearing (58) comprises at least one connecting rod (55), which is connected on the one hand to a bearing element (23, 23') of the support plate (20) and on the other hand to a bearing element (53, 53') of the support device (26).

2. Support according to claim 1,
**characterized in**
**that** the control element (72) adjusts the second bearing (58) in a direction, which is at least substantially at right angles to the support face (62) of the support plate (20).

3. Support according to claim 1 or 2,
**characterized in**
**that** the control element (72) comprises a crank handle (68) for actuating the control element (72).

4. Support according to claim 3,
**characterized in**
**that** the control element (72) comprises a spindle (65), which is actuable by means of the crank handle (68) and converts a rotational motion of the crank handle (68) into a stroke for inclining the support plate (20).

5. Support according to one of claims 1 to 4,
**characterized in**
**that** the support device (26) comprises a third bearing (60) for swivelling the support plate (20) about an axis (61), which is at least approximately at right angles to the support face (62) of the support plate (20).

6. Support according to claim 5,
**characterized in**
**that** a braking device (73, 74) is provided for damping the bearing arrangement of the support plate (20) provided by the third bearing (60).

## Revendications

1. Support (1) pour porter au moins un appareil (2, 3, 4), en particulier pour porter des appareils de visualisation à écran et autres appareils médicaux, comprenant un bras porteur (5) et au moins un dispositif porteur (26) qui est relié au moins indirectement au bras porteur (5), le dispositif porteur (26) étant relié, pour recevoir l'appareil (2, 3, 4), à une plaque de support (20) qui, pour incliner l'appareil (2, 3, 4), est susceptible d'être inclinée au moyen d'un organe de positionnement (72),
dans lequel la plaque de support (20) est montée en rotation dans un premier palier (51) du dispositif porteur (26), ledit premier palier (51) définissant l'axe de rotation (52) autour duquel la plaque de support (20) est susceptible de tourner pour l'inclinaison, et
dans lequel la plaque de support (20) est en outre montée dans un second palier (58) du dispositif porteur (26), ledit second palier (58) étant déplaçable dans l'espace au moyen de l'organe de positionnement (72) pour incliner la plaque de support (20),
**caractérisé en ce que** le second support (58) comprend au moins une bielle (55) qui est reliée d'une part à un élément de palier (23, 23') de la plaque de support (20) et d'autre part à un élément de palier (53, 53') du dispositif porteur (26).

2. Support selon la revendication 1, **caractérisé en ce que** l'organe de positionnement (72) déplace le second palier (58) dans une direction qui est au moins sensiblement perpendiculaire à la surface porteuse (62) de la plaque de support (20).

3. Support selon l'une ou l'autre des revendications 1 et 2, **caractérisé en ce que** l'organe de positionnement (72) comprend une manivelle à main (68) pour actionner l'organe de positionnement (72).

4. Support selon la revendication 3, **caractérisé en ce que** l'organe de positionnement (72) comprend une broche (65) susceptible d'être actionnée au moyen de la manivelle à main (68) et qui convertit un mouvement de rotation de la manivelle à main (68) en une course pour incliner la plaque de support (20).

5. Support selon l'une des revendications 1 à 4, **caractérisé en ce que** le dispositif porteur (26) comprend un troisième palier (60) pour faire basculer la plaque de support (20) autour d'un axe (61) qui est au moins approximativement perpendiculaire à la surface porteuse (62) de la plaque de support (20).

6. Support selon la revendication 5, **caractérisé en ce qu'**il est prévu un dispositif de freinage (73, 74) pour amortir le montage de la plaque de support (20) défini par le troisième palier (60).
